# EUROPEAN PATENT APPLICATION

(11) **EP 4 806 489 A1**
(43) Date of publication of application: **16.09.2026**
(21) Application number: 23958287.7
(22) Date of filing: 09.11.2023
(51) Int. Cl.: A61M 25/09

(54) **MEDICAL DEVICE**

(71) Applicant: ASAHI INTECC CO., LTD., Seto-shi, Aichi, 489-0071 (JP)
(72) Inventor: CHIKAOKA Sora, Seto-shi, Aichi 489-0071 (JP); USHIDA Keisuke, Seto-shi, Aichi 489-0071 (JP); KAWASHIMA Naoya, Seto-shi, Aichi 489-0071 (JP); FUKUI Wataru, Seto-shi, Aichi 489-0071 (JP)
(74) Representative: Winter, Brandl - Partnerschaft mbB
(86) International application number: PCT/JP2023/040314
(87) International publication number: WO 2025/099886

(57) **Abstract**

A medical device includes a first region having a first characteristic, and a second region having a second characteristic and located on the proximal end side of the first region. For example, the medical device includes a specific member containing a nickel-titanium alloy and is located in the first region and the second region. The first characteristic includes the average crystal grain size of the nickel-titanium alloy being a first size, and the second characteristic includes the average crystal grain size of the nickel-titanium alloy being a second size smaller than the first size.

## Description

### TECHNICAL FIELD

The technology disclosed in the present specification relates to a medical device.

### BACKGROUND ART

For example, methods using catheters are widely performed to treat or examine a stenosed portion or an occluded portion (hereinafter referred to as a "lesion portion") in a blood vessel. In order to guide a catheter to the lesion portion in the blood vessel, a guidewire is used. To improve the vessel selectivity of the guidewire, an operator performs a procedure called "shaping", in which the distal end portion of the guidewire is pre-bent at a predetermined angle (see, e.g., Patent Literatures 1 to 3).

### CITATION LIST

### Patent Literature

Patent Literature 1: JP 2015-65979 A
Patent Literature 2: JP 2013-544575 T
Patent Literature 3: JP 2011-125556 A

### SUMMARY OF INVENTION

### TECHNICAL PROBLEM

In a guidewire, it is sometimes required that a first region on the distal end side and a second region located on the proximal end side of the first region exhibit different characteristics. For example, in order to improve the selectivity for fine branching vessels such as peripheral blood vessels, it may be necessary, during shaping, to impart a greater curvature or bending angle to the first region of the guidewire than to the second region (the region on the proximal end side of the first region). However, conventional guidewires have the problem in that the characteristics of the first region and the second region cannot be made different from each other as desired. This problem is not limited to guidewires but is common to medical devices in general.

The present specification discloses a technology capable of solving the above-described problems.

### SOLUTION TO PROBLEM

The technology disclosed in the present specification can be implemented, for example, in the following aspects.
(1) A medical device disclosed in the present specification includes a first region having a first characteristic, and a second region having a second characteristic, the second region being located on the proximal end side of the first region. According to the present medical device, the characteristics of the first region and the second region can be made different from each other as desired.
(2) The medical device may include a specific member containing a nickel-titanium alloy in the first region and the second region. The first characteristic may include the average crystal grain size of the nickel-titanium alloy being a first size, and the second characteristic may include the average crystal grain size of the nickel-titanium alloy being a second size smaller than the first size. According to the present configuration, the bending angle of the first region of the medical device can be made greater than the bending angle of the second region. As a result, for example, shaping in which the bending angle of the first region of the medical device is greater than the bending angle of the second region can be achieved, thereby improving selectivity for fine branching vessels such as peripheral blood vessels.
(3) In the medical device, the first size may be 0.05 µm or more and 2.5 µm or less. According to the present configuration, selectivity for fine branching vessels such as peripheral blood vessels can be improved.
(4) In the medical device, the second size may be 0.01 µm or more and 0.050 µm or less. According to the present configuration, selectivity for fine branching vessels such as peripheral blood vessels can be improved.
(5) In the medical device, the second size may be 0.02 µm or more and 0.048 µm or less. According to the present configuration, selectivity for fine branching vessels such as peripheral blood vessels can be improved.
(6) In the medical device, the first region may include a distal end side first region and a proximal end side first region located on the proximal end side of the distal end side first region, and the average crystal grain size of the nickel-titanium alloy in the distal end side first region may be greater than the average crystal grain size of the nickel-titanium alloy in the proximal end side first region. According to the present configuration, the bending angle of the distal end side first region of the medical device can be made greater than the bending angle of the proximal end side first region. As a result, for example, shaping in which the bending angle of the distal end side first region of the medical device is greater than the bending angle of the proximal end side first region can be achieved, thereby effectively improving selectivity for fine branching vessels such as peripheral blood vessels.
(7) In the medical device, the average crystal grain size of the nickel-titanium alloy in the distal end side first region may be 1.0 µm or more and 2.5 µm or less. According to the present configuration, selectivity for fine branching vessels such as peripheral blood vessels can be effectively improved.
(8) In the medical device, the average crystal grain size of the nickel-titanium alloy in the distal end side first region may be 1.5 µm or more and 2.3 µm or less. According to the present configuration, selectivity for fine branching vessels such as peripheral blood vessels can be effectively improved.
(9) In the medical device, the average crystal grain size of the nickel-titanium alloy in the proximal end side first region may be 0.05 µm or more and 0.1 µm or less. According to the present configuration, selectivity for fine branching vessels such as peripheral blood vessels can be effectively improved.
(10) In the above medical device, the specific member may be a core shaft. According to the present configuration, the bending angle of the first region of the medical device including the core shaft can be made greater than the bending angle of the second region.
(11) In the medical device, when the bending angle of the medical device is measured by pressing the medical device against a flat surface with a pin, applying a load of 1 N to the medical device toward the flat surface via the pin, and then pulling out the medical device from the flat surface in a direction orthogonal to the flat surface, thereby imparting a curved shape to a pulled-out portion of the medical device, the first characteristic may include the bending angle being a first angle, and the second characteristic may include the bending angle being a second angle smaller than the first angle. According to the present configuration, the bending angle of the first region of the medical device can be made greater than the bending angle of the second region. As a result, for example, shaping in which the bending angle of the first region of the medical device is greater than the bending angle of the second region can be achieved, thereby improving selectivity for fine branching vessels such as peripheral blood vessels.
(12) In the medical device, the first angle may be 10 degrees or more and 100 degrees or less. According to the present configuration, selectivity for fine branching vessels such as peripheral blood vessels can be improved.
(13) In the medical device, the second angle may be 0 degrees or more and 20 degrees or less. According to the present configuration, selectivity for fine branching vessels such as peripheral blood vessels can be improved.
(14) In the medical device, the first region may include a distal end side first region and a proximal end side first region located on the proximal end side of the distal end side first region, and the bending angle of the distal end side first region may be greater than the bending angle of the proximal end side first region. According to the present configuration, the bending angle of the distal end side first region of the medical device can be made greater than the bending angle of the proximal end side first region. As a result, for example, shaping in which the bending angle of the distal end side first region of the medical device is greater than the bending angle of the proximal end side first region can be achieved, thereby effectively improving selectivity for fine branching vessels such as peripheral blood vessels.
(15) In the medical device, the bending angle of the distal end side first region may be 40 degrees or more and 100 degrees or less. According to the present configuration, selectivity for fine branching vessels such as peripheral blood vessels can be improved.
(16) In the medical device, the bending angle of the proximal end side first region may be 10 degrees or more and 40 degrees or less. According to the present configuration, selectivity for fine branching vessels such as peripheral blood vessels can be improved.
(17) In the medical device, a value obtained by dividing the bending angle of the distal end side first region by the bending angle of the second region may be 5 or more and 10 or less. According to the present configuration, the bending angle of the distal end side first region of the medical device can be made greater than the bending angle of the second region by a predetermined degree.
(18) In the medical device, a value obtained by dividing the bending angle of the distal end side first region by the bending angle of the proximal end side first region may be 1 or more and 6 or less. According to the present configuration, the bending angle of the distal end side first region of the medical device can be made greater than the bending angle of the proximal end side first region by a predetermined degree.
(19) The medical device may include a core shaft formed of the same material in the first region and the second region. According to the present configuration, damage to the core shaft due to a physical property gap can be prevented.

It should be noted that the technology disclosed in the present specification can be implemented in various forms and may be achieved, for example, as a core shaft for a medical device, a medical device, a method for producing these devices, and the like.

### BRIEF DESCRIPTION OF DRAWINGS

FIG. 1 is an explanatory diagram schematically illustrating a configuration of a guidewire according to an embodiment.
FIG. 2 is an explanatory diagram illustrating a method for determining the average crystal grain size of a nickel-titanium alloy.
FIG. 3 is an explanatory diagram illustrating a method for determining the bending angle.
FIG. 4 is an explanatory diagram illustrating the method for determining the bending angle.
FIG. 5 is an explanatory diagram illustrating a configuration of the guidewire of each modification.
FIG. 6 is an explanatory diagram illustrating a configuration of the guidewire of each modification.

### EMBODIMENTS OF THE INVENTION

### A. Embodiment:

### (Configuration of guidewire 100)

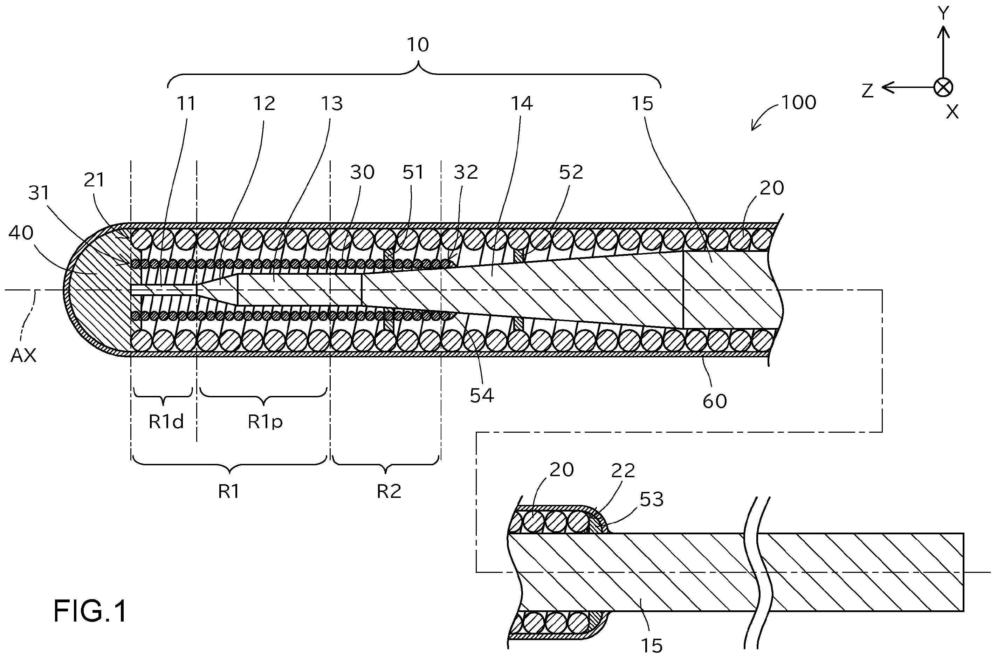

FIG. 1 is an explanatory diagram schematically illustrating a configuration of a guidewire 100 according to an embodiment. In FIG. 1, mutually orthogonal X, Y, and Z axes are shown for defining directions, and a longitudinal cross-section (YZ cross-section) of the guidewire 100 is illustrated. Along the direction parallel to a central axis AX of the guidewire 100 (hereinafter referred to as "axial direction"), a positive side of Z-axis direction corresponds to the distal end side (distal side) on which the guidewire 100 is inserted into the body, and a negative side of Z-axis direction corresponds to the proximal end side (proximal side) on which the guidewire 100 is manipulated by an operator such as a physician. Although FIG. 1 illustrates a state in which the guidewire 100 as a whole has a linear shape substantially parallel to the Z-axis direction, the guidewire 100 has sufficient flexibility to be bent. In the present specification, regarding the guidewire 100 and its constituent components, the end on the distal end side is referred to as "distal end", the distal end and its vicinity are referred to as "distal end portion", the end on the proximal end side is referred to as "proximal end", and the proximal end and its vicinity are referred to as "proximal end portion".

The guidewire 100 is a medical device. The guidewire 100 is inserted into a blood vessel, for example, to guide another medical device such as a catheter (not shown) to a lesion portion in the blood vessel. The guidewire 100 includes a core shaft 10, an outer coil body 20, an inner coil body 30, a distal end side joint portion 40, a first intermediate joint portion 51, a second intermediate joint portion 52, a first proximal end side joint portion 53, a second proximal end side joint portion 54, and a coating 60.

The core shaft 10 is an elongated member. The central axis of the core shaft 10 substantially coincides with the central axis AX of the guidewire 100. The core shaft 10 includes a first portion 11, a second portion 12, a third portion 13, a fourth portion 14, and a fifth portion 15. The first portion 11, the second portion 12, the third portion 13, the fourth portion 14, and the fifth portion 15 are arranged in this order from the distal end toward the proximal end side. The core shaft 10 is an example of the specific member.

In the present embodiment, the first portion 11, the third portion 13, and the fifth portion 15 of the core shaft 10 each have a constant cross-sectional shape (XY cross-section) at each position along the axial direction. The cross-sectional area of the third portion 13 is larger than the cross-sectional area of the first portion 11. The cross-sectional area of the fifth portion 15 is larger than the cross-sectional area of the third portion 13. The second portion 12 and the fourth portion 14 of the core shaft 10 smoothly connect the cross-sectional shapes of other adjacent portions along the axial direction. The second portion 12 and the fourth portion 14 are tapered portions in which the cross-sectional area gradually increases from the distal end side toward the proximal end side.

The core shaft 10 having such a shape can be produced, for example, by performing press processing at a press ratio corresponding to the shape of each portion of the core shaft 10 on a precursor having a constant cross-section along the axial direction.

Examples of a material for forming the core shaft 10 include a metallic material, and more specifically, stainless steel (such as SUS302, SUS304, or SUS316), a nickel-titanium alloy, a piano wire, a nickel-chromium alloy, a cobalt alloy, and tungsten. In the present embodiment, the core shaft 10 includes a nickel-titanium alloy and, more specifically, is formed of a nickel-titanium alloy. In the present embodiment, the entire core shaft 10 is formed of the same material. According to the present configuration, damage to the core shaft 10 due to a physical property gap can be prevented.

The outer coil body 20 is a member formed into a hollow cylindrical shape by helically winding a wire. The outer coil body 20 is, for example, a tightly wound coil. The outer coil body 20 is disposed so as to surround the outer periphery of the distal end portion of the core shaft 10. In the axial direction, the position of a distal end 21 of the outer coil body 20 is substantially the same as the position of the distal end of the core shaft 10.

The inner coil body 30 is a member formed into a hollow cylindrical shape by helically winding a wire. The inner coil body 30 is, for example, a tightly wound coil. The inner coil body 30 is disposed in the space between the core shaft 10 and the outer coil body 20 so as to surround the outer periphery of the distal end portion of the core shaft 10. In the axial direction, the position of a distal end 31 of the inner coil body 30 is substantially the same as the position of the distal end of the core shaft 10, and the position of a proximal end 32 of the inner coil body 30 is on the distal end side of the position of a proximal end 22 of the outer coil body 20.

The outer diameter and the inner diameter of the outer coil body 20 and the inner coil body 30 may be constant along the axial direction, or may vary along the axial direction.

Examples of a material for forming the outer coil body 20 and the inner coil body 30 include a metallic material, and, more specifically, a radiolucent alloy such as stainless steel (such as SUS302, SUS304, or SUS316), a nickel-titanium alloy, a piano wire, a nickel-chromium alloy, or a cobalt alloy, and a radiopaque alloy such as gold, platinum, tungsten, or an alloy containing these elements (e.g., a platinum-nickel alloy).

The distal end side joint portion 40 joins the distal end portion of the core shaft 10 with the distal end portions of the outer coil body 20 and the inner coil body 30. The outer peripheral surface on the distal end side of the distal end side joint portion 40 is a smooth surface (e.g., a substantially hemispherical surface). The first proximal end side joint portion 53 joins the core shaft 10 with the proximal end portion of the outer coil body 20. The second proximal end side joint portion 54 joins the core shaft 10 with the proximal end portion of the inner coil body 30. The first intermediate joint portion 51 joins the core shaft 10 with the intermediate portion (the portion excluding the distal end portion and the proximal end portion; the same applies hereinafter) of the outer coil body 20 and the intermediate portion of the inner coil body 30. The second intermediate joint portion 52 is located on the proximal end side of the first intermediate joint portion 51 and joins the core shaft 10 with the intermediate portion of the outer coil body 20. Examples of a material for forming these joint portions 40, 51, 52, 53, and 54 include metallic solder such as silver solder, gold solder, zinc, an Sn-Ag alloy, or an Au-Sn alloy, and an adhesive such as an epoxy-based adhesive.

The coating 60 covers the outer peripheral surfaces of the distal end side joint portion 40 and the outer coil body 20, extending from the distal end of the distal end side joint portion 40 to the proximal end 22 of the outer coil body 20. Examples of a material for forming the coating 60 include a hydrophilic coating material such as polyvinylpyrrolidone, polyacrylic acid, polyacrylamide, polyvinyl alcohol, a maleic anhydride copolymer, or hyaluronic acid.

### (Characteristic regarding average crystal grain size of nickel-titanium alloy contained in core shaft 10)

The guidewire 100 includes a first region R1 and a second region R2 located on the proximal end side of the first region R1. The first region R1 is a region extending along the axial direction, for example, from the position of the distal end of the core shaft 10 to the position of the intermediate portion of the third portion 13. The first region R1 is a region that has undergone a heat treatment. The second region R2 is a region that has not undergone a heat treatment. The proximal end of the first region R1 is adjacent to the distal end of the second region R2. The length of the second region R2 is predetermined in consideration of the length used by a physician to perform shaping. Along the axial direction, the length of the first region R1 is, for example, 12 mm. The length of the second region R2 is, for example, 5 mm.

As described above, the core shaft 10 contains a nickel-titanium alloy. The average crystal grain size Dₐᵥₑ of the nickel-titanium alloy contained in the core shaft 10 differs between the first region R1 and the second region R2 of the guidewire 100. Specifically, the average crystal grain size Dₐᵥₑ of the nickel-titanium alloy in the second region R2 is smaller than the average crystal grain size Dₐᵥₑ of the nickel-titanium alloy in the first region R1. The average crystal grain size Dₐᵥₑ of the nickel-titanium alloy in the first region R1 is, for example, 0.05 µm or more and 2.5 µm or less. The average crystal grain size Dₐᵥₑ of the nickel-titanium alloy in the second region R2 is, for example, 0.01 µm or more and 0.05 µm or less. The average crystal grain size Dₐᵥₑ of the nickel-titanium alloy in the second region R2 is preferably 0.02 µm or more and 0.048 µm or less. The feature that the average crystal grain size Dₐᵥₑ of the nickel-titanium alloy in the first region R1 is a value (a first value) greater than the average crystal grain size Dₐᵥₑ of the nickel-titanium alloy in the second region R2 is an example of the first characteristic, and the feature that the average crystal grain size Dₐᵥₑ of the nickel-titanium alloy in the second region R2 is a value (a second value) smaller than the average crystal grain size Dₐᵥₑ of the nickel-titanium alloy in the first region R1 is an example of the second characteristic.

The first region R1 of the guidewire 100 includes a distal end side first region R1d and a proximal end side first region R1p located on the proximal end side of the distal end side first region R1d. The distal end side first region R1d is a region extending along the axial direction, for example, from the position of the distal end of the core shaft 10 to the position of the proximal end of the first portion 11. The proximal end side first region R1p is a region extending along the axial direction, for example, from the position of the proximal end of the distal end side first region R1d to the position of the intermediate portion of the third portion 13. The length of the distal end side first region R1d along the axial direction is, for example, 4 mm, and the length of the proximal end side first region R1p is, for example, 8 mm.

The average crystal grain size Dₐᵥₑ of the nickel-titanium alloy in the distal end side first region R1d is greater than the average crystal grain size Dₐᵥₑ of the nickel-titanium alloy in the proximal end side first region R1p. The average crystal grain size Dₐᵥₑ of the nickel-titanium alloy in the distal end side first region R1d is, for example, 1.0 µm or more and 2.5 µm or less. The average crystal grain size Dₐᵥₑ of the nickel-titanium alloy in the distal end side first region R1d is preferably 1.5 µm or more and 2.3 µm or less. The average crystal grain size Dₐᵥₑ of the nickel-titanium alloy in the proximal end side first region R1p is, for example, 0.05 µm or more and 0.1 µm or less.

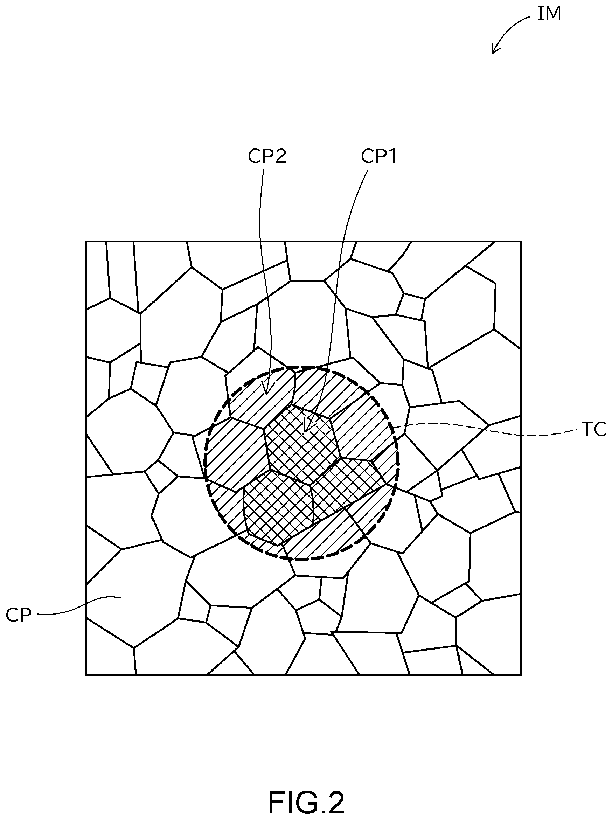

The average crystal grain size Dₐᵥₑ of the nickel-titanium alloy contained in the core shaft 10 in each region of the guidewire 100 is determined as follows. FIG. 2 is an explanatory diagram illustrating a method for determining the average crystal grain size Dₐᵥₑ of the nickel-titanium alloy.

First, a portion near the surface of the core shaft 10 to be measured is cut out as an observation sample. This surface-near portion is a portion that has undergone the heat treatment in the first region R1. The cut-out observation sample is then processed using a focused ion beam (FIB) processing/observation apparatus (e.g., FB-2000 manufactured by Hitachi High-Tech Corp.), and the sample is observed using a transmission electron microscopy (TEM) (e.g., JEM-2100F manufactured by JEOL Ltd.) to obtain a TEM image IM.

Next, in the TEM image IM, a test circle TC having a radius r₀ and an area S₀ (e.g., r₀ = 75 nm) is set, and the number of crystal grains CP included within the test circle TC (the equivalent number of crystal grains N) is counted. Specifically, when the number of crystal grains CP that are entirely included within the test circle TC (e.g., a crystal grain CP1) is defined as N₁, and the number of crystal grains that intersect the test circle TC (e.g., a crystal grain CP2 in FIG. 2) is defined as N₂, the equivalent number of crystal grains N is calculated by the following formula (1). The number of crystal grains per unit area N₀ and the average crystal grain area Sₐᵥₑ are calculated by the following formulas (2) and (3), respectively. The average crystal grain size Dₐᵥₑ is calculated by the following formula (4).
[Mathematical 1] $N = {N}_{1} + \frac{{N}_{2}}{2}$ [Mathematical 2] ${N}_{0} = \frac{N}{{S}_{0}}$ [Mathematical 3] ${S}_{ave} = \frac{1}{{N}_{0}}$ [Mathematical 4] ${D}_{ave} = 2 ⋅ \sqrt{\frac{{S}_{ave}}{π}}$

### (Characteristics regarding bending angle θ)

The first region R1 of the guidewire 100 is more prone to deformation during shaping as compared with the second region R2. That is, regarding a bending angle θ described below, which is an index value representing the tendency to deformation during shaping, the bending angle θ in the first region R1 is greater than the bending angle θ in the second region R2. The bending angle θ in the first region R1 is, for example, 10 degrees or more and 100 degrees or less. The bending angle θ in the second region R2 is, for example, 0 degrees or more and 20 degrees or less. The feature that the bending angle θ in the first region R1 is a value (a first angle) greater than bending angle θ in the second region R2 is an example of the first characteristic, and the feature that the bending angle θ in the second region R2 is a value (a second angle) smaller than bending angle θ in the first region R1 is an example of the second characteristic.

The bending angle θ in the distal end side first region R1d of the guidewire 100 is greater than the bending angle θ in the proximal end side first region R1p. The bending angle θ in the distal end side first region R1d is, for example, 40 degrees or more and 100 degrees or less. The bending angle θ in the proximal end side first region R1p is, for example, 10 degrees or more and 40 degrees or less.

A value obtained by dividing the bending angle θ in the distal end side first region R1d by the bending angle θ in the second region R2 is 5 or more and 10 or less. A value obtained by dividing the bending angle θ in the distal end side first region R1d by the bending angle θ in the proximal end side first region R1p is 1 or more and 6 or less.

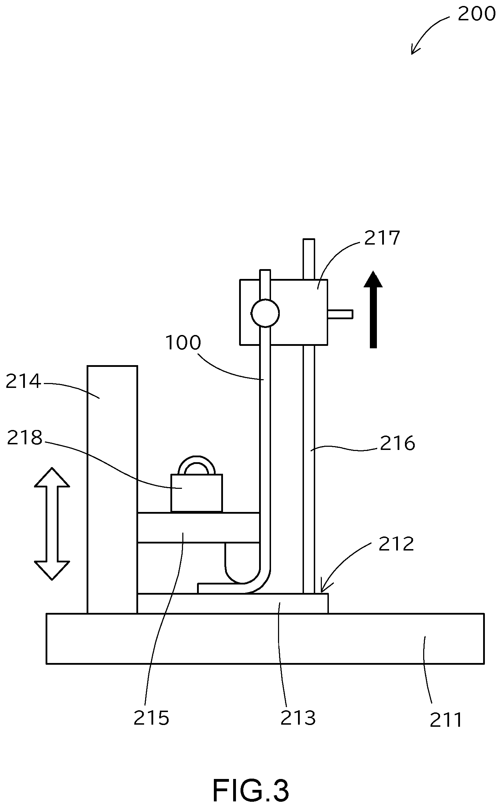

The bending angle θ in each region of the guidewire 100 is determined as follows. FIG. 3 is an explanatory diagram illustrating a method for determining the bending angle θ. FIG. 3 illustrates a measuring device 200 for the bending angle θ. The measuring device 200 includes a base 211, a flat plate 213 placed on the base 211 and having a flat surface 212 as its upper surface, a pin 215 supported so as to be slidable in the vertical direction with respect to a support column 214 erected on the base 211, and a clamp mechanism 217 supported so as to be slidable in the vertical direction with respect to a support rod 216 erected on the base 211.

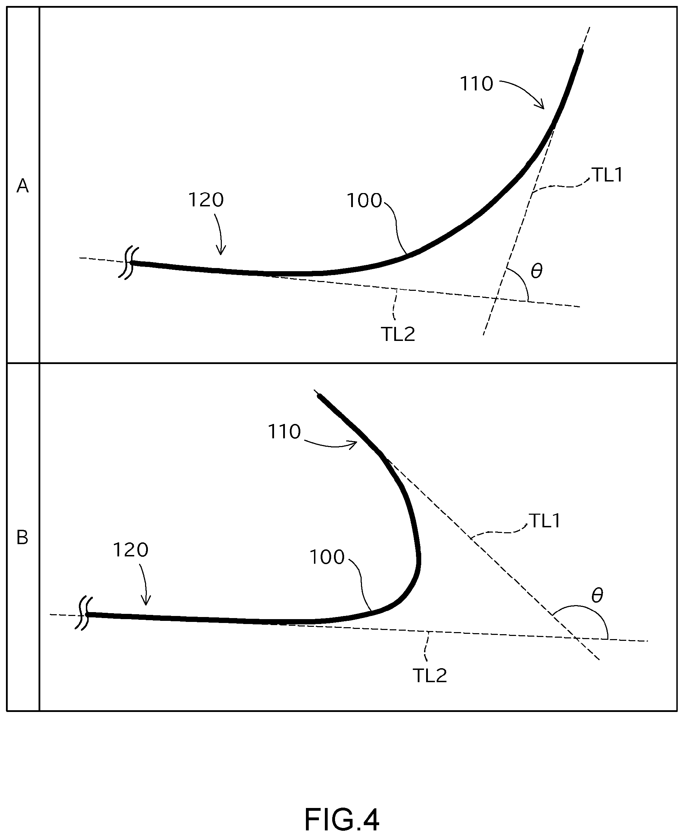

The guidewire 100 to be measured is set on the measuring device 200. Specifically, the guidewire 100 is fixed to the clamp mechanism 217 at a position away from the distal end of the guidewire 100 by a predetermined distance (about 250 mm to 300 mm) so that the guidewire 100 extends downward from the position fixed to the clamp mechanism 217, abuts against the flat surface 212, and extends horizontally on the flat surface 212 from that position. A portion of the guidewire 100 that is in contact with the flat surface 212 is pressed toward the flat surface 212 by the pin 215. By placing a weight 218 on the pin 215, a load of 1 N is applied to the guidewire 100 toward the flat surface 212 via the pin 215. In this state, the clamp mechanism 217 is moved upward along the support rod 216, thereby pulling the guidewire 100 out from the flat surface 212 in a direction orthogonal to the flat surface 212. The amount of pulling is set to about 2 mm. For example, when the bending angle θ of the distal end side first region R1d is to be measured, the bending angle θ is measured by pulling out the central portion of the distal end side first region R1d. As a result, a curved shape is imparted to the pulled-out portion of the guidewire 100. The curved angle at the distal end portion of the guidewire 100 formed in this manner is measured as the bending angle θ. As a method for measuring the bending angle θ, as illustrated in FIG. 4, tangents TL1 and TL2 are drawn to a distal end side portion 110 and a proximal end side portion 120 of the pulled-out portion, respectively, and the angle at which the tangents intersect is determined as the bending angle θ.

### (Method for producing guidewire 100)

For example, by subjecting a portion of the core shaft 10 located in the first region R1 to the heat treatment while not subjecting a portion located in the second region R2 to the heat treatment, it is possible to produce the guidewire 100 in which the average crystal grain size Dₐᵥₑ and the bending angle θ of the nickel-titanium alloy described above differ between the first region R1 and the second region R2. It is likely that, by performing the heat treatment, crystal grains of the nickel-titanium alloy contained in the core shaft 10 become coarser, resulting in an increase in the tendency to deformation, thereby increasing the bending angle θ.

For example, by setting the temperature in the heat treatment applied to the portion of the core shaft 10 located in the distal end side first region R1d to be higher than the temperature in the heat treatment applied to the portion located in the proximal end side first region R1p, it is possible to produce the guidewire 100 in which the average crystal grain size Dₐᵥₑ and the bending angle θ of the nickel-titanium alloy described above differ between the distal end side first region R1d and the proximal end side first region R1p. It is likely that, by performing the heat treatment at a higher temperature, crystal grains of the nickel-titanium alloy contained in the core shaft 10 become further coarsened, resulting in a further increase in the tendency to deformation, thereby further increasing the bending angle θ.

### (Effects of present embodiment)

As described above, the guidewire 100 of the present embodiment includes the first region R1 and the second region R2 located on the proximal end side of the first region R1. The guidewire 100 includes the core shaft 10. The core shaft 10 is located in the first region R1 and the second region R2 and contains the nickel-titanium alloy. The average crystal grain size Dₐᵥₑ of the nickel-titanium alloy contained in the core shaft 10 in the first region R1 is greater than the average crystal grain size Dₐᵥₑ of the nickel-titanium alloy contained in the core shaft 10 in the second region R2. Therefore, according to the guidewire 100 of the present embodiment, characteristics can be differentiated between the first region R1 and the second region R2 as desired. For example, the bending angle θ of the first region R1 of the guidewire 100 can be made greater than the bending angle θ of the second region R2. As a result, shaping can be achieved in which the bending angle θ of the first region R1 of the guidewire is greater than the bending angle θ of the second region R2, thereby improving selectivity for fine branched vessels such as peripheral blood vessels.

In general, in guidewires, the outer diameter of a core shaft may be tapered to become smaller toward the distal end in order to reduce distal end load, or the outer diameter of a coil body may be tapered to become smaller toward the distal end in order to improve penetrability. In such cases, since the bending strain decreases due to a reduction in metal thickness, a shaping feature is obtained in which the curvature or bending angle becomes smaller toward the distal end of the guidewire. Even when the outer diameters of the core shaft and the coil body are made uniform, only a shaping feature having a uniform curvature or bending angle can be obtained. Accordingly, conventional guidewires often have a shaping feature in which the curvature or bending angle becomes smaller toward the distal end, or a shaping feature having a uniform curvature or bending angle. In the guidewire 100 of the present embodiment, as described above, the bending angle θ in the first region R1 of the guidewire 100 can be made greater than the bending angle θ in the second region R2. Therefore, in the guidewire 100, selectivity for fine branching vessels such as peripheral blood vessels can be improved.

In the guidewire 100 of the present embodiment, the first region R1 includes the distal end side first region R1d and the proximal end side first region R1p located on the proximal end side of the distal end side first region R1d. The average crystal grain size Dₐᵥₑ of the nickel-titanium alloy in the distal end side first region R1d is greater than the average crystal grain size Dₐᵥₑ of the nickel-titanium alloy in the proximal end side first region R1p. Thus, according to the guidewire 100 of the present embodiment, the characteristics of the distal end side first region R1d and the proximal end side first region R1p can be made different from each other as desired. More specifically, the bending angle θ of the distal end side first region R1d of the guidewire 100 can be made greater than the bending angle θ of the proximal end side first region R1p. As a result, selectivity for fine branching vessels such as peripheral blood vessels can be effectively improved.

### (Examples)

A worker prepared a plurality of samples of the guidewire 100 of the above-described embodiment. Specifically, regarding the heat treatment of the core shaft 10 used to produce the guidewire 100, the heat treatment was performed on the core shaft 10 in the first region R1. No heat treatment was performed on the core shaft 10 in the second region R2. Using five samples (SA1 to SA5) prepared in this manner, the average crystal grain size Dₐᵥₑ of the nickel-titanium alloy contained in the core shaft 10 was measured. The measurement results are shown in Table 1. In the present examples, the distal end side first region R1d is a region extending from the distal end of the core shaft 10 to a position 4 mm toward the proximal end side, the proximal end side first region R1p is a region extending from the proximal end of the distal end side first region R1d to a position 8 mm toward the proximal end side, and the second region R2 is a region extending from the proximal end of the proximal end side first region R1p to a position 5 mm toward the proximal end side. For the sample SA4, measurement of the distal end side first region R1d was not performed, and for the sample SA5, measurement of the first region R1 was not performed.

**[Table 1]**

| Sample No. | Average crystal grain size (µm) | | |
|---|---|---|---|
| | First region R1 | | Second region R2 |
| | Distal end side first region R1d | Proximal end side first region R1p | |
| SA1 | 1.89 | 0.0938 | 0.0294 |
| SA2 | 2.06 | 0.0686 | 0.0459 |
| SA3 | 2.04 | 0.0870 | 0.0417 |
| SA4 | - | 0.0898 | 0.0426 |
| SA5 | - | - | 0.0385 |

As shown in Table 1, in all of the samples subjected to measurement, the average crystal grain size Dₐᵥₑ of the nickel-titanium alloy in the second region R2 was smaller than the average crystal grain size Dₐᵥₑ of the nickel-titanium alloy in the first region R1. The average crystal grain size Dₐᵥₑ of the nickel-titanium alloy in the first region R1 was 0.05 µm or more and 2.5 µm or less. The average crystal grain size Dₐᵥₑ of the nickel-titanium alloy in the second region R2 was 0.01 µm or more and 0.05 µm or less. More specifically, the average crystal grain size Dₐᵥₑ of the nickel-titanium alloy in the second region R2 was 0.02 µm or more and 0.048 µm or less.

In all of the samples subjected to measurement, the average crystal grain size Dₐᵥₑ of the nickel-titanium alloy in the distal end side first region R1d was greater than the average crystal grain size Dₐᵥₑ of the nickel-titanium alloy in the proximal end side first region R1p. The average crystal grain size Dₐᵥₑ of the nickel-titanium alloy in the distal end side first region R1d was 1.0 µm or more and 2.5 µm or less. More specifically, the average crystal grain size Dₐᵥₑ of the nickel-titanium alloy in the distal end side first region R1d was 1.5 µm or more and 2.3 µm or less. The average crystal grain size Dₐᵥₑ of the nickel-titanium alloy in the proximal end side first region R1p was 0.05 µm or more and 0.1 µm or less.

The bending angle θ was measured for another four samples (SA11 to SA14). The measurement results are shown in Table 2. The samples SA11 to SA13 were prepared according to the production method of the above-described embodiment, whereas the sample SA14 (Comparative example) was prepared such that, regarding the heat treatment of the core shaft 10 used to produce the guidewire 100, no heat treatment was performed in either the first region R1 or the second region R2.

**[Table 2]**

| Sample No. | Bending angle (degrees) | | | Ratio of bending angle | |
|---|---|---|---|---|---|
| | First region R1 | | Second region R2 | R1d/R2 | R1d/R1p |
| | Distal end side first region R1d | Proximal end side first region R1p | | | |
| SA11 | 56 | 17 | 9 | 6.2 | 3.3 |
| SA12 | 69 | 15 | 11 | 6.3 | 4.6 |
| SA13 | 54 | 21 | 6 | 9.0 | 2.6 |
| SA14 | 107 | 115 | 129 | 0.8 | 0.9 |

As shown in Table 2, in the samples SA11 to SA13 (Examples), the bending angle θ in the first region R1 was greater than the bending angle θ in the second region R2. The bending angle θ in the first region R1 was 10 degrees or more and 100 degrees or less. The bending angle θ in the second region R2 was 0 degrees or more and 20 degrees or less. In contrast, in the sample SA14 (Comparative example), the bending angle θ in the first region R1 was smaller than the bending angle θ in the second region R2.

In the samples SA11 to SA13 (Examples), the bending angle θ in the distal end side first region R1d was greater than the bending angle θ in the proximal end side first region R1p. The bending angle θ in the distal end side first region R1d was 40 degrees or more and 100 degrees or less. The bending angle θ in the proximal end side first region R1p was 10 degrees or more and 40 degrees or less. In contrast, in the sample SA14 (Comparative example), the bending angle θ in the distal end side first region R1d was smaller than the bending angle θ in the proximal end side first region R1p.

In the samples SA11 to SA13 (Examples), the values obtained by dividing the bending angle θ in the distal end side first region R1d by the bending angle θ in the second region R2 were 5 or more and 10 or less, and the values obtained by dividing the bending angle θ in the distal end side first region R1d by the bending angle θ in the proximal end side first region R1p were 1 or more and 6 or less.

Using a blood vessel model simulating peripheral blood vessels, the above samples were evaluated for ease of vessel selection. As a result, the samples SA11 to SA13 (Examples) were evaluated as good on ease of vessel selection. In contrast, the evaluation results indicated that the sample SA14 exhibited ease of vessel selection comparable to that of conventional products.

### B. Modifications:

The technology disclosed in the present specification is not limited to the above-described embodiment, and various modifications may be made without departing from the gist thereof. For example, the following modifications are possible.

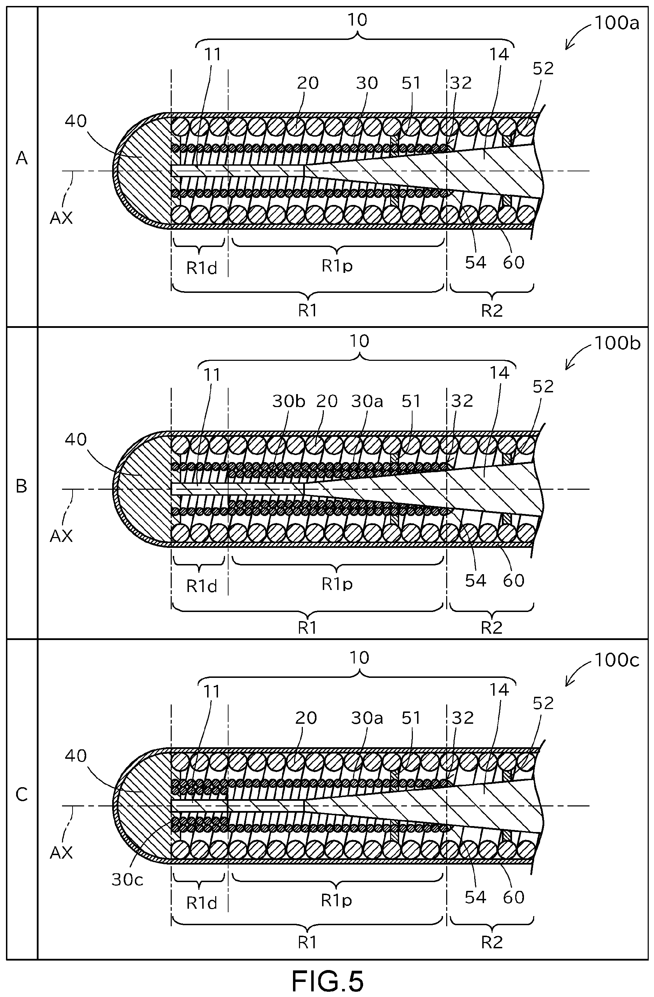

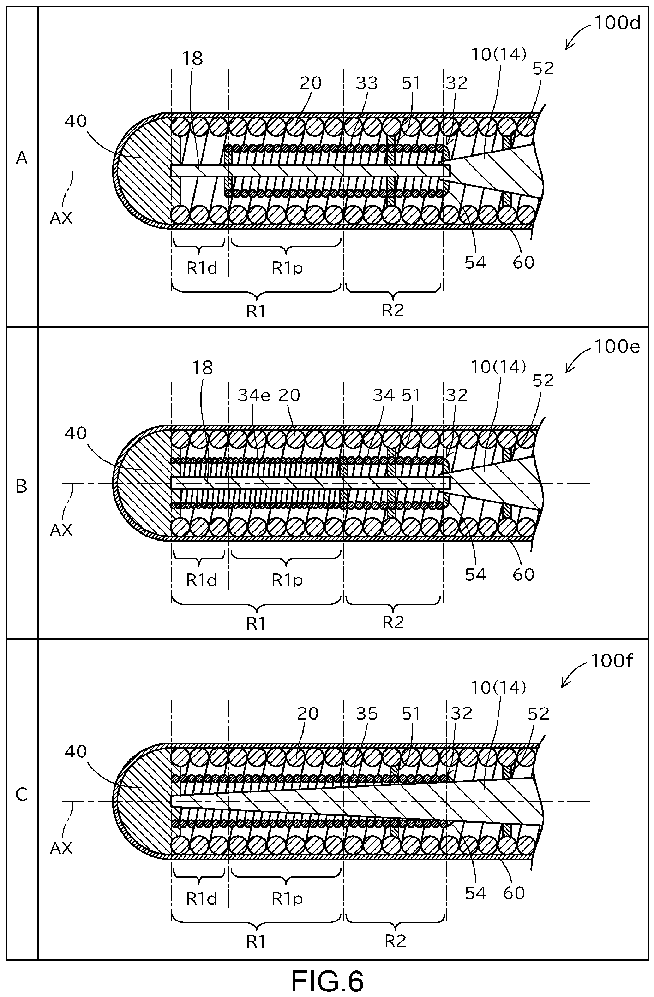

The configuration of the guidewire 100 in the above-described embodiment is merely an example, and various modifications are possible. FIG. 5 and FIG. 6 are explanatory diagrams illustrating the configuration of the guidewire 100 (guidewires 100a to 100f) of modifications.

The guidewire 100a of a first modification shown in column A of FIG. 5, similarly to the guidewire 100 of the above-described embodiment, has a characteristic in which the bending angle θ in the first region R1 is greater than the bending angle θ in the second region R2. However, in the guidewire 100a of the first modification, such a characteristic is achieved not by the configuration of the core shaft 10 but by adopting a configuration in which the tendency to deformation of the coil body increases toward the distal end side. More specifically, the tendency to deformation of the inner coil body 30 in the distal end side first region R1d is greater than the tendency to deformation of the inner coil body 30 in the proximal end side first region R1p. As a result, the bending angle θ in the distal end side first region R1d becomes greater than the bending angle θ in the proximal end side first region R1p. The inner coil body 30 is not disposed in the second region R2. As a result, the bending angle θ in the first region R1 becomes greater than the bending angle θ in the second region R2. The tendency to deformation of the coil body can be adjusted, for example, by changing the temperature of the heat treatment applied to the coil body or by changing the diameter of the wire forming the coil body.

The guidewire 100b of a second modification shown in column B of FIG. 5 adopts an inner coil body 30a that is prone to deformation. Further, a second inner coil body 30b that is less prone to deformation is disposed inside the inner coil body 30a. The proximal end of the second inner coil body 30b is located at the proximal end of the proximal end side first region R1p, and the distal end of the second inner coil body 30b is located at the distal end of the proximal end side first region R1p. By adopting such a configuration, the bending angle θ in the first region R1 becomes greater than the bending angle θ in the second region R2, and the bending angle θ in the distal end side first region R1d becomes greater than the bending angle θ in the proximal end side first region R1p.

The guidewire 100c of a third modification shown in column C of FIG. 5, similarly to the guidewire 100b of the second modification, adopts the inner coil body 30a that is prone to deformation. Further, a third inner coil body 30c that is also prone to deformation is disposed inside the inner coil body 30a. The distal end of the third inner coil body 30c is located at the distal end of the first region R1, and the proximal end of the third inner coil body 30c is located at the proximal end of the distal end side first region R1d. By adopting such a configuration, the bending angle θ in the first region R1 becomes greater than the bending angle θ in the second region R2, and the bending angle θ in the distal end side first region R1d becomes greater than the bending angle θ in the proximal end side first region R1p.

The characteristic in which the bending angle θ in the first region R1 is greater than the bending angle θ in the second region R2 may also be achieved by adopting a configuration in which the tendency to deformation is uniformly imparted to the core shaft 10, and the restoring force of the coil body decreases toward the distal end side.

The guidewire 100d of a fourth modification shown in column A of FIG. 6 adopts a configuration in which the tendency to deformation is uniformly imparted to the core shaft 10, and the restoring force of the coil body decreases toward the distal end side. Specifically, the distal end of an inner coil body 33 is located at the distal end of the proximal end side first region R1p. The restoring force of the inner coil body 33 in the proximal end side first region R1p is smaller than the restoring force of the inner coil body 33 in the second region R2. By adopting such a configuration, the bending angle θ in the first region R1 becomes greater than the bending angle θ in the second region R2, and the bending angle θ in the distal end side first region R1d becomes greater than the bending angle θ in the proximal end side first region R1p. In the guidewire 100d of the fourth modification, the distal end portion of the core shaft 10 is formed as a separate body from the other portion and is constituted by a ribbon body 18 fixed to the distal end of the other portion. The ribbon body 18 may be formed of a material (e.g., stainless steel) different from the material forming the other portion of the core shaft 10 (e.g., a nickel-titanium alloy). The same applies to the following fifth modification.

The guidewire 100e of a fifth modification shown in column B of FIG. 6, similarly to the guidewire 100d of the fourth modification, adopts a configuration in which the tendency to deformation is uniformly imparted to the core shaft 10, and the restoring force of the coil body decreases toward the distal end side. Specifically, the distal end of an inner coil body 34 is located at the distal end of the second region R2. A fourth inner coil body 34e having a smaller outer diameter than the inner coil body 34 is disposed in the first region R1. The restoring force of the fourth inner coil body 34e in the distal end side first region R1d is smaller than the restoring force of the fourth inner coil body 34e in the proximal end side first region R1p. The restoring force of the fourth inner coil body 34e is smaller than the restoring force of the inner coil body 34. By adopting such a configuration, the bending angle θ in the first region R1 becomes greater than the bending angle θ in the second region R2, and the bending angle θ in the distal end side first region R1d becomes greater than the bending angle θ in the proximal end side first region R1p.

The guidewire 100f of a sixth modification shown in column C of FIG. 6 adopts a configuration in which the tendency to deformation is uniformly imparted to an inner coil body 35, and the restoring force of the core shaft 10 decreases toward the distal end side. Specifically, the distal end portion of the core shaft 10 has a tapered shape in which the diameter increases toward the proximal end side. Accordingly, the outer diameter of the core shaft 10 in the proximal end side first region R1p is smaller than the outer diameter of the core shaft 10 in the second region R2, and the outer diameter of the core shaft 10 in the distal end side first region R1d is smaller than the outer diameter of the core shaft 10 in the proximal end side first region R1p. By adopting such a configuration, the bending angle θ in the first region R1 becomes greater than the bending angle θ in the second region R2, and the bending angle θ in the distal end side first region R1d becomes greater than the bending angle θ in the proximal end side first region R1p.

In the above-described embodiment, the numerical ranges and relative sizes of the average crystal grain size Dₐᵥₑ of the nickel-titanium alloy contained in the core shaft 10 in each region of the guidewire 100 are merely examples, and various modifications may be made.

In the above-described embodiment, the numerical ranges and relative sizes of the bending angle θ in each region of the guidewire 100 are merely examples, and various modifications may be made.

In the above-described embodiment, the characteristics imparted to each region (the first region R1 and the second region R2) of the guidewire 100 are merely examples, and other characteristics may be imparted.

The technology disclosed in the present specification is not limited to the guidewire 100 and is applicable, for example, to percutaneous medical devices in general, including dilators. Further, the technology disclosed in the present specification is applicable not only to percutaneous medical devices but also to medical devices in general.

## Claims

1. A medical device (100) comprising:
a first region (R1) having a first characteristic; and
a second region (R2) having a second characteristic, the second region (R2) being located on a proximal end side of the first region (R1).

2. The medical device (100) according to claim 1, comprising a specific member (10) containing a nickel-titanium alloy, the specific member (10) being located in the first region (R1) and the second region (R2), wherein:
the first characteristic includes an average crystal grain size of the nickel-titanium alloy being a first size; and
the second characteristic includes the average crystal grain size of the nickel-titanium alloy being a second size smaller than the first size.

3. The medical device (100) according to claim 2, wherein the first size is 0.05 µm or more and 2.5 µm or less.

4. The medical device (100) according to claim 2 or 3, wherein the second size is 0.01 µm or more and 0.050 µm or less.

5. The medical device (100) according to any one of claims 2 to 4, wherein the second size is 0.02 µm or more and 0.048 µm or less.

6. The medical device (100) according to any one of claims 2 to 5, wherein the first region (R1) includes a distal end side first region (R1d) and a proximal end side first region (R1p) located on a proximal end side of the distal end side first region (R1d), the average crystal grain size of the nickel-titanium alloy in the distal end side first region (R1d) being greater than the average crystal grain size of the nickel-titanium alloy in the proximal end side first region (R1p).

7. The medical device (100) according to claim 6, wherein the average crystal grain size of the nickel-titanium alloy in the distal end side first region (R1d) is 1.0 µm or more and 2.5 µm or less.

8. The medical device (100) according to claim 6 or 7, wherein the average crystal grain size of the nickel-titanium alloy in the distal end side first region (R1d) is 1.5 µm or more and 2.3 µm or less.

9. The medical device (100) according to any one of claims 6 to 8, wherein the average crystal grain size of the nickel-titanium alloy in the proximal end side first region (R1p) is 0.05 µm or more and 0.1 µm or less.

10. The medical device (100) according to any one of claims 2 to 9, wherein the specific member (10) is a core shaft (10).

11. The medical device (100) according to any one of claims 1 to 10, wherein: when a bending angle of the medical device (100) is measured by pressing the medical device (100) against a flat surface (212) with a pin (215), applying a load of 1 N to the medical device (100) toward the flat surface (212) via the pin (215), and then pulling out the medical device (100) from the flat surface (212) in a direction orthogonal to the flat surface (212), thereby imparting a curved shape to a pulled-out portion of the medical device,
the first characteristic includes the bending angle being a first angle; and
the second characteristic includes the bending angle being a second angle smaller than the first angle.

12. The medical device (100) according to claim 11, wherein the first angle is 10 degrees or more and 100 degrees or less.

13. The medical device (100) according to claim 11 or 12, wherein the second angle is 0 degrees or more and 20 degrees or less.

14. The medical device (100) according to any one of claims 11 to 13, wherein the first region (R1) includes a distal end side first region (R1d) and a proximal end side first region (R1p) located on a proximal end side of the distal end side first region (R1d), the bending angle in the distal end side first region (R1d) being greater than the bending angle in the proximal end side first region (R1p).

15. The medical device (100) according to claim 14, wherein the bending angle in the distal end side first region (R1d) is 40 degrees or more and 100 degrees or less.

16. The medical device (100) according to claim 14 or 15, wherein the bending angle in the proximal end side first region (R1p) is 10 degrees or more and 40 degrees or less.

17. The medical device (100) according to any one of claims 14 to 16, wherein a value obtained by dividing the bending angle in the distal end side first region (R1d) by the bending angle in the second region (R2) is 5 or more and 10 or less.

18. The medical device (100) according to any one of claims 14 to 17, wherein a value obtained by dividing the bending angle in the distal end side first region (R1d) by the bending angle in the proximal end side first region (R1p) is 1 or more and 6 or less.

19. The medical device (100) according to any one of claims 11 to 18, comprising a core shaft (10) located in the first region (R1) and the second region (R2), the core shaft (10) being formed of a same material.
